# NEW EUROPEAN PATENT SPECIFICATION

(11) **EP 1 964 544 B2**
(45) Date of publication and mention of the opposition decision: **02.08.2023**
(45) Mention of the grant of the patent: 26.07.2017
(21) Application number: 08152107.2
(22) Date of filing: 28.02.2008
(51) Int. Cl.: A61K 8/33, A61K 8/34, A61K 8/35, A61K 8/37, A61Q 13/00, C11D 3/00

(54) **Sensitive skin perfumes**
Parfüms für empfindliche Haut
Parfums pour peaux sensibles

(30) Priority: 02.03.2007 EP 07290269
(43) Date of publication of application: 03.09.2008
(73) Proprietor: Takasago International Corporation, Tokyo 144-8721 (JP)
(72) Inventor: Warr, Jonathan, 75017 Paris (FR); Fraser, Stuart, Little Neston, Cheshire CH64 4DH (GB); Grouault, Olivier, 92320 Chatillon (FR)
(74) Representative: Cabinet Beau de Loménie

(56) References cited:
- EP-A- 1 504 744
- EP-A- 1 661 978
- EP-A1- 0 004 732
- WO-A-00/27197
- WO-A-97/15283
- WO-A-98/56888
- WO-A-02/074430
- WO-A1-89/00042
- US-A- 5 676 163
- US-A1- 2003 008 802
- US-A1- 2005 003 975

## Description

### Technical Field of the Invention

The invention relates to simple well characterised fragrance compositions for use in cosmetic, toiletry, personal care and personal cleansing and household and laundry products which help to reduce allergic reactions and provides information because every ingredient can be identified on the product packaging.

### Background of the invention

Perfumes in cosmetic, toiletry, personal care and cleansing, household cleaning and freshening and laundry products provide several functions. They mask base odours, provide an olfactory aesthetic benefit and serves as a signal of product attributes and function, e.g. hygiene, cleanliness, mildness etc. Unfortunately as well as these benefits, perfumes can also be the cause of allergic reactions in a small proportion of the population.

One solution adopted by some manufacturers is to manufacture fragrance free products as in US2006/110466. Notwithstanding the unpleasant nature of the allergic reaction experienced, some suffers still prefer to use fully fragranced products. There have been many other approaches to prevent or ameliorate the effects of allergic reactions. US patent 5,297,732 describes a fragranced container containing an unfragranced product. Other patents describe the addition of ingredients to formulations to minimize allergic reactions to the product rather than specifically concentrating on the fragrance, e.g. US patent 5,653,997 describes the use of curcurbitine in skin cream to inhibit histamine formation.

Different routes to reduce or prevent allergic reactions which deal with the fragrance compositions have already been proposed, for example:
- US2005/119156 describes the synthesis of new molecules with similar olfactory characteristics as known allergens, while EP1657294 claims that blending fragrances with two types of antioxidant will prevent the formation of certain peroxides known to be potent allergens and thereby reduce allergic reactions.
- EP 0251644 describes the stereospecific synthesis and use in fragrances of the L isomer of 3,7 - dimethyl-7-hydroxyoctilidene anthranilate which has lower allergic reaction potential than the corresponding D isomer.

Despite these efforts there is still a need for consumer products fragranced in a way that can either avoid causing allergic reactions for a greater proportion of the population and which provides sufficiently detailed fragrance compositional information on the pack so that users can avoid materials to which they react.

### Summary of the Invention

The present invention relates to a fragrance composition which may be used in cosmetic, toiletry, personal care and cleansing, household cleaning and laundry products. Said composition consists of 2 to 10 well characterized fragrance materials having a cosmetic function, of which at least 4 are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, 2-t-butylcyclohexylacetate and pentyl salicylate,
and must comprise at least 40% by weight of the composition, wherein the well characterized fragrance materials having a cosmetic function are selected from Table 1 below.

Optionally the fragrance composition of the invention may also contain natural extracts.

According to another aspect of the invention, all or part of the fragrance composition may be present in an encapsulated form.

According to yet another aspect of the invention is the use of these perfume compositions and encapsulated perfume compositions in cosmetic, toiletry, personal care and cleansing, household cleaning and laundry products.

### Detailed Description of the invention

### Well Characterized Fragrance Materials

In the context of this specification a "well characterized fragrance material", which term is synonymous with a "well characterized perfume material" or "well characterized perfume or fragrance ingredient" is an essential part of the invention.

For the purpose of the invention, the expression "well characterized fragrance material having a cosmetic function" means a fragrance having an INCI name (defined by the International Nomenclature of Cosmetic Ingredients), which has purity greater than 90% by weight as defined below and has a cosmetic function.

In commercial fragrance ingredients it is not possible to achieve total purity and even among different batches of the same material from the same supplier it is possible for the levels of minor compounds to vary. Isomerisation, rearrangement, hydrogenation and dehydrogenation are examples of chemical changes, which can occur during manufacture and materials of this type structurally related to the major compounds are not considered to be impurities. Materials of this type which are structural, geometric or optical isomers or which differ by no more than 2 mass units from the major compound, specifically when one single bond is replaced by a double bond or vice versa having the same carbon skeleton and functional groups other than saturation/unsaturation are not considered to be impurities. For the purpose of the invention, well characterized fragrance ingredients of the current invention may contain up to 10% by weight of other materials and even more preferably they contain less than 1% by weight of other materials.

Well characterized fragrance ingredients may result from chemical synthesis using available commercial chemicals, or by purification of natural materials, or by chemical reactions on a naturally derived raw material or from microbial biosynthesis. Well characterized fragrance ingredients may well comprise mixtures. These mixtures can include different chemical species, such as starting materials together with reaction products and/or mixtures of isomers, i.e. compounds having the same chemical formula and therefore the same molecular weight. Isomers may be structural which means that compounds have the same chemical formula and molecular weight but the atoms are joined together in a different order. Thus, n-pentanol and isopentanol are structural isomers and for the purposes of this specification these are considered as the same ingredient. Stereoisomers have atoms joined in the same order but with a different spatial arrangement and can be subdivided into geometric isomers or optical isomers. Thus alpha amyl cinnamic aldehyde exists as cis-and trans-stereoisomers of the double bond, which are considered here as a single ingredient. Optical isomers are molecules which cannot be superimposed on their mirror images. While most naturally derived compounds are single isomers, many chemical reactions generate mixtures of optical isomers and such mixtures are considered single chemical species for the purposes of this specification.

Perfume ingredients are described more fully in S. Arctander, Perfume Flavors and Chemicals. Vols. I and II, Montclair, N.J., and the Merck Index, 8th Edition, Merck & Co., Inc. Rahway, N.J., both are incorporated herein by reference. However it is well known that some fragrance materials can be harmful, irritating the skin or sensitive areas such as the eyes or cause an allergic reaction in sensitized subjects. Perfume manufacturers are careful in their use of fragrance ingredients to comply with guidelines laid down by industry bodies such as The International Fragrance Research Association (IFRA) and relevant national legislation.

A category of fragrance materials which are particularly safe across a wide variety of product applications and dosage levels are those which have a cosmetic function. These ingredients are well established either through widespread use over along period of time or more recent introductions which are supported by extensive safety testing.

For the purpose of the invention, well characterized fragrance materials having a cosmetic function can be defined as those fragrance materials that are used for other purposes within a cosmetic, toiletry or personal care formulation as described in Commission Decision of European Communities 2006/257/EU of February 9, 2006 amending Decision 96/335/EC, which is subject to amendments and corrections.

EU Commission Decision 96/335/EC established an inventory and a common nomenclature of ingredients employed in cosmetic products (INCI) and was amended by Decision 2006/257/EC.

The Cosmetic, Toiletry and Fragrance Association periodically publishes up-dated list of INCI materials. The most recent edition is International Cosmetic Ingredient Dictionary and Handbook, 11th Ed. (2006).

In the Commission Decision of European Communities 2006/257/EU, the cosmetic functions of ingredients are listed and defined in its annex 1, section 1, which is incorporated herein by reference. The well characterized fragrance ingredients of the invention are also listed in annex 1, section 1, and have an International Nomenclature of Cosmetic Ingredients (INCI) name. Well characterized fragrance ingredients having the following cosmetic functions are preferred for the preservative compositions of the invention: stabilizing, emollient, viscosity controlling, antifoaming, solvent, hydrotrope, skin conditioning, emulsifying, toning, denaturing, refreshing, UV filter, UV absorbing, humectant, antistatic, antioxidant, deodorant, hair drying, hair conditioning, antimicrobial, preservative, plasticizer, soothing and masking.

Particularly preferred fragrance ingredients are those which have the following cosmetic functions: stabilizing, emollient, viscosity controlling, antifoaming, solvent, hydrotrope, skin conditioning, emulsifying, soothing toning, refreshing, UV filter, UV absorbing, humectant, antistatic, antioxidant and deodorant.

Without wishing to be limited, the well characterized perfume ingredients having a cosmetic function of the inventive composition will preferably have molecular weights of less than 325 atomic mass units, preferably less than 300 atomic mass units and more preferably less than 275 atomic mass units to be sufficiently volatile to be perceived. Furthermore the perfume compounds will have preferably molecular weights greater than 70 atomic mass units, preferably greater than 100 atomic mass units as lower masses may be too volatile or too water soluble to function as perfumes. Well characterized perfume ingredients can be found in S. Arctander, Perfume Flavors and Chemicals. Vols. I and II, Montclair, N.J.

Well characterized perfume ingredients of the preservative compositions will not contain strongly ionizing functional groups such as sulphonates, sulphates, or quaternary ammonium ions, nor will they contain any halogen atoms.

Table 1 below lists a number of preferred well characterized fragrance materials which have cosmetic functions.

A particularly preferred list of well characterized fragrance ingredients are those included in table 2 below

**TABLE 1: Preferred well characterized fragrance materials having a cosmetic function**

| **Compound** | **Cas no** | **INCI name** | **Other material (%) by weight** | **Cosmetic function** |
|---|---|---|---|---|
| Acetanilide | 103-84-4 | Acetanilid | Less than 1% | Stabilising |
| Pentyl salicylate | 2050-08-0 | Amyl salicylate | Less than 1% | Skin conditionner |
| Hexanol | 111-27-3 | Hexyl alcohol | Less than 1% | Antifoaming/solvent/hydrotrope |
| Octanol | 111-87-5 | Caprylic alcohol | Less than 1% | Viscosity controlling |
| Aldehyde C-14 (undecalactone gamma) | 104-67-6 | Gamma-undecalactone | Less than 1% | Solvent/masking |
| Aldehyde C-16 (Ethyl methyl phenyl glycidate) | 77-83-8 | Ethyl methylphenyl Glycidate | Less than 1% | Viscosity controlling |
| Aldehyde C-18 (nonalactone, gamma) | 104-61-0 | Gamma-nonalactone | Less than 1% | Solvent |
| Allyl caproate | 123-68-2 | Allyl caproate | Less than 1% | Emollient |
| Amyl acetate | 628-63-7 | Amyl acetate | Less than 1% | Solvent |
| Amyl benzoate | 2049-96-9 | Amyl benzoate | Less than 1% | Solvent/masking |
| Benzyl acetate | 140-11-4 | Benzyl acetate | Less than 1% | Solvent |
| Benzaldehyde | 100-52-7 | Benzaldehyde | Less than 1% | Solvent |
| Benzyl salicylate | 118-58-1 | Benzyl salicylate | Less than 1% | UV absorber |
| Benzyl alcohol | 100-51-6 | Benzyl alcohol | Less than 5% | Preservative/solvent |
| Isopropyl myristate | 11-21-0 | Isopropyl myristate | Less than 5% | Binding/emollient/solvent/skin conditioner |
| Citronellol | 106-22-9 | Citronellol | Less than 5% | Masking |
| Citronellyl acetate | 150-84-5 | Citronellyl acetate | Less than 5% | Masking |
| Costaulon ^{®} | 67770-79-0 | Ethyl butylvalerolactone | Less than 1% | Tonic |
| Coumarin | 91-64-5 | Coumarin | Less than 1% | Masking |
| Cyclamen aldehyde | 103-95-7 | Cyclamen aldehyde | Less than 1% | Masking |
| Cyclopentadecanolide | 106-02-5 | Pentadecalactone | Less than 1% | Masking |
| Decalactone | 705-86-2 | Delta-decalactone | Less than 1% | Masking |
| Decanal | 112-31-2 | Decanal | Less than 5% | Masking |
| Dihydro isojasmonate | 37172-53-5 | Dihydrojasmonate | Less than 1% | Tonic |
| Dipropylene glycol | 25265-71-8 | Dipropylene glycol | Less than 10% | Solvent |
| Efetaal ^{®} | 2556-10-7 | Ethyl phenethylacetal | Less than 1% | Solvent/tonic |
| Ethyl cinnamate | 103-36-6 | Ethyl cinnamate | Less than 1% | UV absorber |
| Ethyl laurate | 106-33-2 | Ethyl laurate | Less than 1% | Emollient |
| Ethyl pelargonate | 123-29-5 | Ethyl pelargonate | Less than 1% | Emollient |
| Ethyl vanillin | 121-32-4 | Ethyl vanillin | Less than 1% | Soothing/masking |
| Fleuramone ^{®} | 137-03-1 | 2-Heptylcyclopentanone | Less than 1% | Solvent |
| Geraniol | 106-24-1 | Geraniol | Less than 1% | Tonic |
| Geranyl acetate | 105-87-3 | Geranyl acetate | Less than 5% | Tonic |
| Geranyl acetate | 105-87-3 | Geranyl acetate | Less than 5% | Tonic |
| Heliotropine | 120-57-0 | Heliotropine | Less than 1% | Masking/skin conditioning |
| Cis hex-3-en-1-ol | 928-96-1 | 3-Hexenol | Less than 1% | Solvent/humectant/ viscosity controlling |
| Hydroxycitronellal | 107-75-5 | Hydroxycitronellal | Less than 1% | Masking |
| Iso butyl benzoate | 120-50-3 | Isobutyl benzoate | Less than 1% | Preservative/solvent |
| Linalool | 78-70-6 | Linalool | Less than 5% | Deodorant |
| Linalyl acetate | 115-95-7 | Linalyl acetate | Less than 5% | Masking |
| Menthyl acetate | 89-48-5 | Menthyl acetate | Less than 1% | Refreshing/masking |
| Methyl dihydrojasmonate | 2630-39-9 | Methyldihydro-jasmonate | Less than 1% | Masking |
| Methyl salicylate | 119-36-8 | Methyl salicylate | Less than 1% | Denaturing/soothing |
| Ethylene brassylate | 105-95-3 | Ethylene brassylate | Less than 10% | Tonic/masking |
| Phenoxanol | 55066-48-3 | Phenylisohexanol | Less than 1% | Masking |
| Phenoxyethanol | 122-99-6 | Phenoxyethanol | Less than 1% | Preservative |
| Phenyl salicylate | 118-55-8 | Phenyl salicylate | Less than 5% | Antimicrobial |
| Triacetin | 102-76-1 | Triacetin | Less than 1% | Antimicrobial/film forming /solvent |
| Propyl acetate | 109-60-4 | Propyl acetate | Less than 1% | Solvent |
| Santalex T^{®} | 68877-29-2 | Camphylcyclohexanol | Less than 10% | Solvent |
| Triethyl citrate | 77-93-0 | Triethyl citrate | Less than 1% | Antioxidant/ deodorant/solvent |
| Vanillin | 121-33-5 | Vanillin | Less than 1% | Masking |
| Verdox^{®} | 88-41-5 | 2-t-butylcyclohexylacetate | Less than 1% | Solvent |
| 2-Phenyl ethanol | 60-12-8 | Phenylethanol | Less than 5% | Deodorant |
| 2-Phenylethyl acetate | 103-45-7 | Phenyl ethyl acetate | Less than 5% | Deodorant |
| Anethol | 104-46-1 | Anethol | Less than 1% | Denaturant |
| Benzophenone | 119-61-9 | Benzophenone | Less than 1% | UV absorber |
| Citronellal | 106-23-0 | Citronellal | Less than 5% | Masking agent |
| Decenal | 65405-70-1 | Decenal | Less than 5% | Masking agent |

| **Compound** | **CAS no** | **INCI name** | **Other material (%) by weight** | **Cosmetic function** |
|---|---|---|---|---|
| Methyl anthranilate | 134-20-3 | Methyl anthranylate | Less than 1% | Masking |
| Paracymene | 99-87-6 | P-cymene | Less than 5% | Masking |
| Terpineol | 98-55-5 | Terpineol | Less than 10% | Denaturant/solvent |
| Hinokitiol | 499-44-5 | Hinokitiol | Less than 1% | Antistatic/hair conditioning |

**TABLE 2: Particularly Preferred Well Characterized Fragrance Materials Having a Cosmetic Function**

| **Compound** | **CAS n°** | **INCI name** | **Cosmetic function** |
|---|---|---|---|
| Aldehyde C-14 (undecalactone gamma) | 104-67-6 | Gamma-undecalactone | Solvent |
| Aldehyde C-16 (ethyl methyl phenyl glycidate) | 77-83-8 | Ethyl methylphenyl glycidate | Viscosity controlling |
| Aldehyde C-18 (nonalactone, gamma) | 104-61-0 | Gamma-nonalactone | Solvent |
| Allyl caproate | 123-68-2 | Allyl caproate | Emollient |
| Benzyl acetate | 140-11-4 | Benzyl acetate | Solvent |
| Benzaldehyde | 100-52-7 | Benzaldehyde | Solvent |
| Dihydroisojasmonate | 37172-53-5 | Dihydrojasmonate | Tonic |
| Ethyl cinnamate | 103-36-6 | Ethyl cinnamate | UV absorber |
| Ethyl vanillin | 121-32-4 | Ethyl vanillin | Soothing |
| Fleuramone ^{®} | 137-03-1 | 2-heptylcyclopentanone | Solvent |
| Geranyl acetate | 105-87-3 | Geranyl acetate | Tonic |
| Heliotropine | 120-57-0 | Heliotropine | Skin conditioning |
| Cis hex-3-en-1-ol | 928-96-1 | 3-hexenol | Solvent/humectant/ viscosity controlling |
| Hinokitiol | 499-44-5 | Hinokitiol | Antistatic |
| Ethylene brassylate | 105-95-3 | Ethylene brassylate | Tonic |
| 2-phenylethanol | 60-12-8 | Phenylethanol | Deodorant |
| 2-phenylethylacetate | 103-45-7 | Phenylethylacetate | Deodorant |
| Santalex T^{®} | 68877-29-2 | Camphylcyclohexanol | Solvent |
| Verdox | 88-41-5 | 2-t-butylcyclohexylacetate | Solvent |
| Pentyl salicylate | 2050-08-0 | Amyl salicylate | Skin conditionner |
| Efetaal^{®} | 2556-10-7 | Ethyl phenethylacetal | Solvent/tonic |

Fragrances for consumer products usually require a large number of fragrance ingredients to perform satisfactorily. Typically commercial fragrances may contain from 20 to 200 individual ingredients. While it is known to use a single material as a product fragrance the result is usually inferior to a fully formulated fragrance. As the number of fragrance ingredients increases so does the creative scope.

Surprisingly it has been found that consumer desirable fragrances can be formulated from groups of 2 to 10 ingredients. So for fragrance quality at least two or more well characterised fragrance ingredients having a cosmetic function are required, preferably more than 3 or 4 ingredients and especially preferably more than 5 ingredients. There is no distinct upper limit; however the requirement to keep formulations simple enough to be listed on the pack does require a limit. Moreover the increase in creative scope on increasing from 2 to 4 ingredients is much greater than in going from 10 to 12 ingredients.

Surprisingly it has been found that attractive and successful fragrances can be created from as few as two well characterized fragrance ingredients and a range of attractive successful fragrances having different fragrance notes can be created from 4 to 7 well characterised fragrance ingredients having a cosmetic function. Thus it seems reasonable to limit the maximum number of ingredients to 10 or less, preferably 9 ingredients or less, more preferably 8 ingredients or less and especially preferably 7 ingredients or less.

The fragrance composition of the present invention contains at least 2 and at most 10 well characterized fragrance materials having a cosmetic function, of which at least 4 are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate,
and said at least 4 well characterized fragrance materials constitute at least 40% by weight of the composition. As above mentioned, the fragrance composition of the present invention may also contain natural extracts, such as essential oils.

Within the fragrance composition at least 40% by weight and preferably 50% by weight of the fragrance must comprise at least 4 well characterised fragrance materials chosen from among allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate

### Exclusions and Limitations

The invention specifies precise numbers of well characterized fragrance ingredients and some fragrance ingredients may not be chemically pure compounds eg natural extracts and essential oils which may be variable in composition from different sources, methods of extraction, even seasonal variations. Hence for the purposes of defining the invention essential oils and natural extracts are excluded from the definition of well characterized fragrance ingredients having a cosmetic function, although it is recognized that some essential oils may have beneficial effects. It is also well known that essential oils and various extracts are widely used in toiletry and personal care products as fragrance ingredients or for the many other benefits they provide thus essential oils are optional ingredients of the invention.

Among the perfumery materials which although permitted as well characterized fragrance materials are known to have undesirable characteristics and are therefore preferably excluded from the invention perfume compositions are nitro musks as exemplified by musk ketone (CAS 81-14-1) are preferably excluded due to concerns of human safety. Carbitol ethers defined as compounds of formula R-(OCH₂CH₂)ₙ -OR¹ where n= 1,2 or 3 R= (C₁ to C₇) alkyl or phenyl or alkyl substituted phenyl and R¹ is H or (C₁ to C₇)alkyl are preferably excluded due to concerns of human safety.

Phthalate esters especially the esters of low molecular weight alcohols such as dimethyl phthalate, diethyl phthalate, dibutyl phthalate are also excluded from the invention fragrance compositions due to concerns of human safety.

There are a few fragrance materials which are well characterized fragrance materials according to the invention definition but which are also known to be strong allergens from prior art publications. (See for example Contact Dermatitis vol. 50, pp 65-76 (2004) and ibid vol. 49, pp 236-240 2003.) These ingredients are: iso eugenol (CAS 97-54-1), cinnamic aldehyde (CAS 104-55-2), cinnamic alcohol (CAS 104-54-1), Amyl cinnamic aldehyde (CAS 122-40-7), citral (CAS 5392-40-5), Eugenol (CAS 97-53-0), farnesol (CAS4602-84-0), lilial (CAS 80-54-6) and coumarin (CAS 91-64-5); none of which should be intentionally added to fragrance compositions of the current invention.

### Fragrance level

Fragrance dosage depends on the type of product and some typical dosage levels are shown in table 3 below. Indeed it may be preferred if the fragrance is dosed at lower levels than in conventional fragrances typically below 1.0% by weight of the final product composition, preferably below 0.6% by weight of the final product composition, more preferably below 0.4% by weight of the final product composition and particularly preferably below 0.2% by weight of the final product composition.

### Optional Natural Extracts

Compositions of the present invention may optionally contain natural extracts, such as essential oils. Natural extracts are produced by subjecting suitable natural materials such as plant components: leaves, flowers, seeds, roots or stems to an extraction process. The extraction processes are well known to those skilled in the art and are described in The Essential Oils by E Guenther published in 1949 by D van Nostrand. Essential oils can undergo additional processes to rectify and purify the oils for example by removing the terpene components via a "head cut" and/or removing the wax components via a "tail cut". Such natural extracts include but are not limited to those obtained from citrus species such as: lemons, oranges, mandarin, grapefruit, ugli fruit, anise, clove, basil, aniseed, cinnamon, geranium, roses, mint, lavender, lavandin, thyme, rosemary, citronella, cypress, eucalyptus, peppermint, Peru balsam, camphor, sandalwood, ylang and cedarwood and mixtures thereof. A preferred group of natural extracts for the present invention are Amyris oil, cedarwood oil, cocoa absolute, copaiba balsam, menthe oil pays, myrrh resin, patchouli oil, vanillin (absolute) and vetiver oil.

### Encapsulating material

In a preferred embodiment of the invention, the perfume composition is encapsulated.

A wide variety of capsules exist which will allow for delivery of perfume at various times during or subsequent to use of the products.

Examples of such capsules with different encapsulated materials are capsules provided by microencapsulation. One method comprises a capsule core which is coated completely with a material which may be polymeric. US patent. 4,145,184, and US patent 4,234,627, teach using a tough coating material which essentially prohibits the perfume diffusing out of the capsule. The perfume is delivered to fabric via the microcapsules and is then released by rupture of the micropcapsules such as would occur on ironing or manipulation of a fabric.

In general, preferred encapsulating materials of the perfumed particles can be either a water-insoluble material or a water-soluble encapsulating material. The latter is obviously suited to dry products with the perfume being released when the product is contacted with water, for example an underarm product which will release fragrance on sweating or a powder detergent which releases perfume in contact with water. The water insoluble capsules are more suited to liquid products such as body washes liquid or fabric softeners, with the contents being released by mechanical abrasion.

Suitable water soluble encapsulating materials are capsules which consist of a matrix of polysaccharide and polyhydroxy compounds such as described in GB 1,464,616. Still other suitable water soluble or water dispersible encapsulating materials comprise dextrins derived from ungelatinized starch acid-esters of substituted dicarboxylic acids such as described in U.S. Pat. No. 3,455,838. These acid-ester dextrins are preferably prepared from such starches as waxy maize, waxy sorghum, sage, tapioca and potato.

When starch is employed, the starches suitable for encapsulating the perfume oils of the present invention can be made from raw starch, pregelatinized starch, modified starch derived from tubers, legumes, cereal and grains, for example corn starch, wheat starch, rice starch, waxy corn starch, oat starch, cassava starch, waxy barley, waxy rice starch, sweet rice starch, amioca, potato starch, tapioca starch, oat starch, cassava starch, and mixtures thereof.

Modified starches suitable for use as the encapsulating matrix in the present invention include hydrolyzed starch, acid thinned starch, starch esters of long chain hydrocarbons, starch acetates, starch octenyl succinate, and mixtures thereof.

Modified starches having emulsifying and emulsion stabilizing capacity such as starch octenyl succinates have the ability to entrap the perfume oil droplets in the emulsion due to the hydrophobic character of the starch modifying agent. The perfume oils remain trapped in the modified starch until dissolved in the wash solution.

Suitable examples of said encapsulating materials are N-Lok^{®}, manufactured by National Starch, Narlex^{®},and Capsul ^{®}.These encapsulating materials comprise pregelatinised waxy maize starch and optionally, glucose. The starch may be modified by adding monofunctional substituted groups such as octenyl succinic acid anhydride.

### Manufacture of modified starch encapsulated perfume composition

The following is a non-limiting example of a suitable process for manufacture of a modified starch encapsulated perfume composition for use in cosmetic toiletry personal cleaning and care and household and laundry products according to the present invention.
1. 225 g of CAPSUL modified starch (National Starch & Chemical) is added to 450 g of water at 24°C;
2. The mixture is agitated at 600 RPM (turbine impeller 2 inches in diameter) for 20 minutes;
3. 75 g perfume composition is added near the vortex of the starch solution;
4. The emulsion formed is agitated for an additional 20 minutes (at 600 RPM);
5. Upon achieving a perfume droplet size of less than 15 microns, the emulsion is pumped to a spray drying tower and atomized through a spinning disk with co-current airflow for drying. The inlet air temperature is set at 205-210°C, the exit air temperature is stabilized at 98-103°C;
6. Dried particles of the starch encapsulated perfume composition are collected at the dryer outlet.

Still another preferred manufacture of modified starch encapsulated perfume composition is described in GB 1,464,616, which comprises a mixture of polysaccharide material which is a modified starch and a polyhydroxy compound present in an amount of at least 20% of the mixture by weight and selected from alcohols such as sorbitol, plant-type sugars, lactones, monoethers and acetals. The process comprises forming a solution of the modified starch and the polyhydroxy compound, in proportions such that their mixture softens at the temperature of spray-drying, in water, emulsifying the oil in solution and spray drying said emulsion to remove water therefrom.

Other suitable matrix materials and process details are disclosed e.g. in US patent 3,971,852, which is incorporated herein by reference.

Of course, mixtures of free perfume compositions and encapsulated perfume compositions, which may have the same or different compositions, can be employed in the products of the invention. This will allow a desirable immediate release of fragrance upon opening of the package and a boost of fragrance intensity or release of a different fragrance note at some stage while using the product.

### Water insoluble capsules

Nonlimiting examples of water-insoluble capsule coating materials include polyethylenes, polyamides, polystyrenes, polyisoprenes, polycarbonates, polyesters, polyacrylates, vinyl polymers and polyurethanes and mixtures thereof. A preferred form of water insoluble capsule is an aminoplast capsule formed by condensation polymerization. Various patents describe compositions and processes for manufacturing aminoplast capsules in the form of dispersions such as EP 1,246,693 A1 and US 6,261,483 which are incorporated herein by reference. Without wishing to limit the patent in any way a typical process for preparing a capsule dispersion would include the following steps:
1. the preparation of an emulsion of the perfume ingredients and any other ingredients which may include emulsifying agents or emulsion stabilizers takes place often under vigorous agitation;
2. a melamine:formaldehyde:methanol mixture is added in the approximate molar ratios of 1:3:2 to 1:6:4. These monomers may be precondensed or the monomers may be used directly. Some of the melamine can be replaced by urea if desired;
3. acid is added to adjust to a pH of 3.5 to 6.5 and the reaction temperature raised to 30-45°C and allowed to proceed until the dispersion is oil free. Any acid which has no adverse properties may be used in this process, such as for example formic acid;
4. it is particularly advantageous if a further addition of urea, melamine or other amines e.g. diethanolamine or mixtures thereof can be made to reduce the formaldehyde concentration in the finished dispersion, and increase the wall thickness;
5. it is particularly advantageous if the capsules are cured by heating to a temperature comprised between 60°C to 80°C for several hours.

Capsules of the above process will generally have a particle size within the range from 5-100 µm depending on the emulsifying conditions. The capsule wall will have a thickness of 0.025-1.0 µm.

The final dispersion may typically contain from 2.5%-80%, capsules dispersed in water with the dispersion containing 10%-60% perfume. In some forms of the process excess water can be removed to form either a concentrated wet cake or a dry free flowing powder as best suits the subsequent application.

### Products

Products to be fragranced according to the invention, also named final products in the present description, are personal care, household and laundry compositions which are not intended for human or animal ingestion and especially those products which are to be described as skin mild or for sensitive skin. Included within the definition of products to be ingested for purposes of the present invention are products for dental and oral care, such as toothpastes and mouth washes which although not intended for ingestion may nevertheless accidentally enter the gastro-intestinal tract.

Personal care compositions include creams, emulsions, lotions gels and oils for the skin (face, hands, feet etc) tinted bases (liquids and pastes) deodorant and antiperspirant products, products for removing make-up from the face and eyes.

Hair care products include: hair tints and bleaches, products for waving, straightening, setting and fixing hair.

Shaving products including creams, foams mousses and depilatory products.

Sun bathing products and products for tanning without the sun. Personal cleansing products for the skin include toilet soaps, deodorant soaps, bath and shower preparations (salts, foams, lotions, liquids oils, gels etc.). Hair cleansing products include shampoos and conditioners.

Household cleansing preparations include hand dishwash liquids, general purpose cleansers, liquids, mousses, creams and abrasive liquids.

Laundry compositions include detergent products such as detergent powders tablets and bars, liquid detergents, liquid fine wash products any of which may optionally contain bleach and or enzymes and non deterging fabric treatment products which include fabric softeners, fabric conditioners, tumble drier sheets and ironing waters.

Many of the products will contain a certain proportion of water and such products will usually contain some surface active material, either as an emulsifier, if the product is an emulsion, or as a detergent active material if the product has some kind of cleaning function. For cleaning products the concentration of surface active material in the product will be 0.1-60% by weight; usually the level of surface active material will be 50% by weight or lower; for most products the level of surface active material will be 30% by weight or lower. On the other hand, the level of surface active material will usually be at least 0.1% by weight preferably greater than 1.0% and more preferably greater than 3.0% by weight. For products which have a cleaning function it is likely the level of surface active material will be higher, typically greater than 10% by weight and preferably greater than 15% by weight.

Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make up application or removal, or to apply sunscreen compounds or sunless tanning agents, for personal cleansing e.g. as moist toilet tissue or baby wipes.

Examples of cleansing products containing detergents are: shampoos, body washes, dishwashing liquids, laundry liquids, general purpose cleaners, liquid abrasive cleaners, liquid soaps, laundry detergent powders, detergent bars, fabric softeners and tumble drier sheets. Again articles or substrates such as pads, sponges or wipes made from non woven textiles, may be impregnated with liquids for cleaning inanimate surfaces such as kitchen worktops, tiled surfaces, bathroom sanitaryware, windows, leather goods and soft furnishings.
Some products may fall into more than one category. Typical quantities of water, surface active material and perfume, listed as weight percentage, in different kinds of products are set out in table 3 below.

**Table 3**

| **Product** | **Surface active material (%)** | **Water (%)** | **Perfume (%)** |
|---|---|---|---|
| Oil-in-water skin cream | 10 | 60 | 0.2 |
| Water-in-oil skin cream | 2 | 60 | 0.4 |
| Eye make-up remover | 5 | 60 | 0.2 |
| Liquid abrasive cleaner | 12 | 32 | 0.3 |
| General purpose cleaner | 8 | 90 | 0.3 |
| Shampoo | 20 | 75 | 0.5 |
| Body Wash | 15 | 80 | 1.0 |
| Cleansing Wipes* | 5 | 90 | 0.2 |
| Lotion skin Wipes | 5 | 80 | 0.1 |
| Window cleaner | 0.2 | 90 | 0.1 |
| Fabric softener | 5 | 94 | 0.4 |
| Hair conditioner | 5 | 90 | 0.5 |
| Dishwashing liquid | 40 | 55 | 0.2 |
| Heavy laundry liquid | 15 | 55 | 0.4 |
| Detergent powder | 10-25 | 5-8 | 0.2-0.6 |
| Detergent Bar | 25 | 20 | 0.3 |
| ironing water | 0-1 | 98-99 | 0.05-0.2 |
| * figures based on composition of liquid used to impregnated the wipe | | | |

The formulations and ingredients of household, laundry and personal care products and cosmetics in which preservative compositions of the invention may be used are well known to those skilled in the art, reference may be made to the following works which are incorporated herein by reference:
Formulating Detergents and Personal Care Products A guide to Product Development by L Ho Tan Tai, ISBN 1-893997-10-3 published by the AOCS Press, and
also to Volume 67 of the Surfactant Science Series Liquid Detergents ISBN 0-8247-9391-9 (Marcel Dekker Inc) and Surface Active Agents and Detergents Vols 1 and 2 by Schwartz, Perry and Birch, and Harry's Cosmeticology published by CHS Press 8th Edn 2000 ISBN 0820603724, and McCutcheon's Detergents and Emulsifiers Published by Allured.

Hair and Hair Care Ed by Dale H Johnson ISBN0-8247-9365-X published by Marcel Dekker, as well as to the following patents or patent applications; all of these patents and the references therein are incorporated herein by reference.
Fabric softeners, conditioners and post wash treatments:
   US6,335,315; US5,674,832; US5,759,990; US5,877,145; US5,574,179; US4767547; US6806248; EP1,141,188; EP1,287,198; EP0,459,822; EP0,392,606.
Liquid Laundry detergents:
   US5,929,022; US5,916,862; US5,731,278; US5,470,507; US5,466,802; US5,460,752 and US5,458,810 and WO2001/0053754; WO2006/053615.
Shampoos and Hair Conditioners:
   US6,162,423; US5,968,286; US5,935,561; US5,932,203; US5,837,661; US5,776,443; US5,756,436; US5,661,118; US5,618,523; US2004/021912; EP0018717; EP1009365; EP0200305.
Wipes
WO2003051327; WO0004230, EP1,361,855; US2005/0008680;
Skin cleansing liquids
EP1066827; EP1510201; EP573229; US2005/0085405;

The present compositions are used in a conventional manner for cleaning the skin and/or hair and to provide olfactory aesthetic benefit. An effective amount of the product, typically from about 1 g to about 15 g of the composition, is applied to the body or hair that has preferably been wetted, generally with water. Application includes dispensing of the composition onto the hand, onto the body or hair, or onto a washing implement, e.g. wash cloth, sponge, etc., and typically includes working the composition with the hands to develop lather. The lather can stand on the body for a length of time or can be rinsed immediately with water. Once the product is rinsed from the body the washing procedure can be repeated

Liquid compositions incorporating fragrances of the invention can be dispersed on a tissue, a wipe, towel, towelette, and the like. The material may be flushable. As used herein, by "flushable" is meant that the material will pass through at least 10 feet of waste pipe in two toilet flushes. The material may also be biodegradable.
Materials that can be used can be mono or multi-layered, woven or non woven. They can be made of one or of several materials.
Preferred are non-woven materials that have a web structure of fibrous or filamentous nature, in; which the timbres or filaments are distributed randomly or with a certain degree of orientation, the former being obtainable by air-laying or by certain wet-laying processes, the latter by other wet-laying or by carding processes. The timbres or 5 filaments can be natural, for example wood pulp, wool, cotton, linen and the like, or synthetic, for example polyvinyls, polyesters, polyolefines, polyamides and the like.

Typically they have a basis weight in the range of 10 to 80 g/m2, in particular of 40 to 70 g/m2. Particularly preferred materials are of the non-woven type. Based on the raw material that has been used, two different types of products can be distinguished. o A first type of carrier is paper based. The raw materials for these carriers are made almost exclusively of cellulose-based fibres or filaments from plant sources (pulp). These can be available from fresh wood-shavings or from recycled material (recycled paper). In a number of wipe applications, such as baby wipes, wipes for cleansing, feminine hygiene wipes, wet paper towels and the like, high wet strength or firmness of the non-woven web is a desirable attribute. This can be achieved by the addition of binding materials. Examples of such materials are the so-called wet strength resins. In some cases further additives are incorporated in order to increase the softness of the end product.

In a second type the carrier web is made mainly of staple fibres, e.g. based on cotton, wool, linen synthetic fibres and the like.

Commercial products are made of cellulose fibres, synthetic fibres or mixtures of both. Polyester and polypropylene are known as suitable polymers for the preparation of synthetic fibres. Also in these products, binders can be used to increase the firmness of the non-woven fabric. Webs of increased strength can be obtained by using the so-called spunlace or hydro-entanglement technique. In this technique the individual fibres are twisted together so that an acceptable strength or firmness is obtained without using binding materials. An advantage of the latter technique is the excellent softness of the non-woven material.

Non woven materials that are made of a mixture of pulp and staple are also known. Such materials are available with binding materials, in particular those mentioned above, or without binding materials. In the latter instance the non-woven is preferably made by the spunlace or hydro-entaglement procedure.

The substrates are wetted with a liquid composition. These can be water-based formulations, in particular they can take the form of aqueous solutions or emulsion-based. These emulsion compositions, which are also referred to as 'lotions', preferably are of aqueous nature.

The emulsions can be oil-in-water or water-in-oil emulsions, or be of more complex nature such as water-in-oil-in-water. The emulsions may be made by methods known in the art, including the known phase inversion technique which is preferred for making fine droplet emulsions. Examples and manufacturing processes for phase inversion emulsions are described in WO00/004230.

Aqueous solutions or emulsions of containing fragrances of the invention are dosed onto the substrates at a rate between 100g/m⁻² and 175gm⁻² of substrate.

The invention will now be explained further by the following non limiting examples except the following examples 5 and 11 which are reference examples.

### Example 1

**Fragrance A** is a fragrance composition according to the invention

| **Ingredient** | **% (by weight)** |
|---|---|
| Allyl caproate | 1.0 |
| Dihydrojasmonate | 18.0 |
| Dipropylene Glycol | 25.0 |
| Geranyl acetate | 6.0 |
| Heliotropine | 14.0 |
| Phenoxyethanol | 16.0 |
| Ethylene brassylate | 20.0 |

### Example 2

**Fragrance B** is a fragrance composition according to the invention

| **Ingredient** | **% (by weight)** |
|---|---|
| Benzaldehyde | 1.0 |
| Dihydrojasmonate | 20 |
| Dipropylene Glycol | 33 |
| Cis hex-3 en-1-ol | 1 |
| Santalex T | 13.2 |
| Ethylene brassylate | 30 |
| Iso propyl myristate | 1.8 |

### Example 3

**Fragrance C** is a floral rose fragrance according to the invention which is suited for a liquid or powder detergent product, or a fabric conditioner.

| **Ingredient** | **% (by weight)** |
|---|---|
| Ethyl pelargonate | 3 |
| Ethyl vanillin | 5 |
| Heliotropine | 10 |
| Phenoxyethanol | 19 |
| Santalex T | 17.6 |
| Ethylene brassylate | 27 |
| Iso propyl myristate | 2.4 |
| Terpineol | 16 |

### Example 4

**Fragrance D** is a floral jasmine fragrance according to the invention which is suited for a general purpose household cleaner.

| **Ingredient** | **% (by weight)** |
|---|---|
| Benzyl acetate | 30 |
| Dihydroisojasmonate | 20 |
| Cis Hex-3-en-1-ol | 1 |
| Iso amyl acetate | 19 |
| Santalex T | 8.8 |
| Gamma undecalactone | 10 |
| Ethylene brassylate | 10 |
| Iso propyl myristate | 1.2 |

### Example 5

**Fragrance E** is a sandalwood fragrance according to the invention containing an essential oil suitable for use in liquid or powder detergents and household products.

| **Ingredient** | **% (by weight)** |
|---|---|
| Amyris oil | 15 |
| Ethyl vanillin | 5 |
| Ethylene Brassylate | 30 |
| Santalex T | 44 |
| Isopropyl myristate | 6 |

### Example 6

**Fragrance F** is a fruity fragrance suitable for encapsulation in either water soluble spray dried starch capsules or water insoluble formaldehyde melamine aminoplast capsules. Such capsules can be applied in any of the product categories of the invention.

| **Ingredient** | **% (by weight)** |
|---|---|
| Iso amyl acetate | 30 |
| Ethylene Brassylate | 10 |
| Santalex T | 8.8 |
| Terpineol | 10 |
| Gamma undecalactone | 20 |
| Verdox | 20 |
| Isopropyl myristate | 1.2 |

### Example 7

**Fragrance G** is a floral jasmine fragrance according to the invention which is suitable for toiletry applications.

| **Ingredient** | **% (by weight)** |
|---|---|
| Benzyl acetate | 20 |
| Heliotropine | 10 |
| Cis hex-3-en-1-ol | 1 |
| Ethylene brassylate | 29 |
| Phenoxyethanol | 20 |
| Santalex T | 8.8 |
| Isopropyl myristate | 1.2 |
| Undecalactone gamma | 10 |

### Example 8

**Fragrance H** is a fruity pear fragrance according to the invention which is suitable for personal care products.

| **Ingredient** | **% (by weight)** |
|---|---|
| Amyl salicylate | 50 |
| Dihydroisojasmonate | 10 |
| Ethyl laurate | 10 |
| Iso amyl acetate | 20 |
| Gamma undecalactone | 10 |

### Example 9

**Fragrance I** is a powdery almond fragrance according to the invention which is suitable for personal care products.

| **Ingredient** | **% (by weight)** |
|---|---|
| Benzaldehyde | 10 |
| Ethyl vanillin | 10 |
| Heliotropine | 20 |
| Ethylene brassylate | 60 |

### Example 10

Fragrance J is a floral fragrance according to the invention which is particularly suitable for personal care products such as a shower gel

| **Ingredient** | **% (by weight)** |
|---|---|
| Amyl salicylate | 28 |
| Ethyl cinnamate | 10 |
| Ethyl methyl phenyl glycidate | 40 |
| Fleuramone | 20 |
| Efetaal | 2 |

### Example 11

Fragrance K is a floral fragrance according to the invention which is particularly suitable for personal care products such as baby products

| **Ingredient** | **% (by weight)** |
|---|---|
| Hinokitiol | 2 |
| Ethyl cinnamate | 10 |
| Ethyl methyl phenyl glycidate | 15 |
| Phenylethyl acetate | 10 |
| Phenyl Ethyl Alcohol | 63 |

### Examples 12 to 14

Examples 12 to 14 are conventional low bulk density powders having different builders whilst example 15 is a high bulk density powder generally known as concentrated powders and example 16 is a softening in the wash detergent powder. A second perfume may be post dosed directly onto the detergent powder, and this may be the same fragrance as in the capsule as in examples 12 to 16, but the fragrance may also have a different composition and odour.

| | **Ex. 12** | **Ex. 13** | **Ex. 14** | **Ex. 15** | **Ex. 16** |
|---|---|---|---|---|---|
| Sodium Linear C11-C13 alkyl benzene sulphonate (Na-LAS) | 8.5 | 11 | 11 | 8 | 3.0 |
| Sodium C12-C15 alkyl 3-ethoxy sulphate (AES) | | | | 1.5 | |
| Alcohol ethoxylate Neodol 23 7EO supplied by Shell | 6.5 | 3.5 | 3.5 | 5 | |
| Cationic Praepaqen HY supplied by Clariant | | | | 1.3 | 1.5 |
| Dequest 2060 supplied by Monsanto | | | | | 0.6 |
| Sodium linear C12-C18 Carboxylates | 2 | 1 | 1.2 | 0.3 | |
| Zeolite A24 | 19.5 | | | | |
| Zeolite A4 | | 22 | | 20 | 15.0 |
| Sokolan CP5 supplied by BASF | 1.7 | 3 | | 1 | 2.0 |
| Polyacrylate (mw 5000) | | | 3.5 | | |
| Sodium citrate/citric acid | 2.5 | | 1.5 | 4 | 2 |
| Sodium silicate | 1.5 | | | | 4.0 |
| Sodium disilicate (SKS-6) | | 2.5 | 3.5 | 11 | |
| Sodium carbonate | 18.5 | 18.5 | 28 | 14 | 14.0 |
| Sodium sulphate | 27.5 | 10 | 23 | 4 | |
| Sodium Carboxymethyl Cellulose | 0.15 | | | 0.15 | 0.4 |
| Perfume capsules containing fragrance F of example 6 | 0.26 | 0.26 | 0.26 | 0.39 | |
| Minors | | | | | |
| POST Dosed Ingredient* | | | | | |
| Na LAS | | | | | 3.0 |
| Sodium linear C16-C18 primary alcohol sulphate (Na-PAS) | | | 3.5 | 5.5 | 1.0 |
| Alcohol ethoxylate | | | | | 2.0 |
| Zeolite A | | | | | 8.0 |
| Silicone antifoam (15% active material) | 1 | 0.7 | 0.7 | 1 | 5 |
| Perfume F of Example 6 | 0.15 | 0.15 | 0.15 | 0.25 | 0.3 |
| Sodium Percarbonate | 7 | | 13 | 13 | |
| Tetra-acetylethylene diamine (83% active material) | 1 | 4 | 3.5 | 5 | 6.0 |
| Sodium perborate tetra hydrate | | 19 | | | 12.0 |
| Fluorescer Tinopal CBS-X supplied by Ciba (15% active material) | 1 | 0.7 | 0.7 | 0.7 | |
| Dequest 2047 and 2016 supplied by Monsanto | 0.8 | 0.8 | 1.5 | 1.5 | |
| Enzymes (protease, lipase, cellulase, amylase) | 0.3 | 1 | 1 | 1.5 | 2.6 |
| Speckles (coloured carbonate) | | 1.5 | | | |
| Fabric care polymers (soil or waxy solid release, dye transfer etc) | 0.2 | 0.4 | 0.5 | 1 | |
| Bentonite clay | | | | | 10.0 |
| Moisture + minors | To 100 | To 100 | To 100 | To 100 | To 100 |
| * Other ingredients included in the spray dried powder may also be post dosed as suits a particular manufacturing process or to achieve particular powder properties | | | | | |

### Examples 17-24 Liquid Detergent Compositions

Examples 17 to 20 are standard liquid detergents, while examples 21 to 24 concentrated liquid detergents, all contain fragrances of the invention some encapsulated, some free fragrance and some a mixture of the two.

**Table 7: Liquid Laundry Detergent Compositions**

| Ingredient | Ex 17 st liq | Ex 18 st liq | Ex 19 st liq | Ex 20 st liq | Ex 21 conc liq | Ex 22 conc liq | Ex 23 conc liq | Ex 24 conc liq |
|---|---|---|---|---|---|---|---|---|
| | Wt % | Wt % | Wt % | Wt % | Wt % | Wt % | Wt % | Wt % |
| Na-LAS | 9.5 | | | | 14 | | | |
| Na-PAS | | 4 | 10 | 7 | 4 | 5 | 5 | 15 |
| Na-AES | | | 2 | 2 | | | | 2.6 |
| Nonionic 7EO | 15 | 9 | 3.5 | 4.5 | 15 | 24 | 22 | 5 |
| Cationic | | | 1 | | | | | 2 |
| Soap | 15 | 15 | 7 | 6 | 12 | 17 | 18 | 11 |
| APG | | 4 | | | | 4 | 2.5 | |
| Glucosamide | | | 4.5 | 4 | | | | 6 |
| Mono ethanol amine | | | 5 | 3.5 | | | | 6.5 |
| Sodium citrate | 1 | 1 | 2 | 1.2 | 6 | 1 | 4 | 2 |
| Propylene glycol | | 3 | 6 | 6 | 5 | | 3 | 8 |
| Glycerol | | | | | | 4 | | |
| Ethanol | 7 | 2 | 1 | 1 | | 7 | 1.5 | 2.2 |
| Perfume C of example 3 | 0.3 | 0.2 | 0.2 | 0.4 | 0.5 | 0.5 | 0.4 | 0.6 |
| Aminoplastencapsulated perfume F of example 6 | 0.1 | 0.2 | 0.3 | | 0.1 | 0.2 | 0.3 | |
| Viscosity modifier | 0.25 | | 0.25 | 0.25 | 0.18 | | | 0.18 |
| Anti-foaming agent (15% active material) | 1 | 0.7 | 0.7 | 0.7 | 1 | 1 | 1 | 1 |
| Fluorescence agent (15% active material) | 1.1 | 0.7 | 0.7 | 0.7 | 1 | 1 | 1 | 1 |
| Defloculating and sequestrating agent (Dequest^{®} 2040 and 2010) | 1 | 1 | 1.4 | 1 | 1.5 | 1.5 | 1.5 | 1.5 |
| Enzymes (protease, lipase, cellulase, amylase | 0.7 | 1.05 | 1.05 | 0.9 | 1.2 | 1.2 | 1.2 | 1.2 |
| Polymers for fabric maintenance (elimination of stains dye transfer, etc) | 1 | 1 | 1 | 1 | 1.5 | 1.5 | 1.5 | 1.5 |
| Water | To 100 | To 100 | To 100 | To 100 To 100 | | To 100 | To 100 | To 100 |

### Example 25

Example 25 is a Shampoo Formulation incorporating fragrance I of example 9.

| **Ingredient** | **Wt %** |
|---|---|
| Lauryl Ether Sulphate | 14.0 |
| Cocoamidopropyl betaine | 6.5 |
| Glycerol | 2.0 |
| Sodium N-cocoylamidoethyl N-ethoxycarboxymethylglycinate | 2/0 |
| Coconut Monoethanolamide | 0.8 |
| Copolymer of dimethyldiallyl ammonium chloride and acrylamide | 1.5 |
| Copolymer of acrylic acid and stearyl methacrylate | 0.3 |
| Salicylic Acid | 0.2 |
| Sodium Benzoate | 0.5 |
| Disocdium Ethylene diamine tetra-acetate | 0.25 |
| Perfume I of example 9 | 0.2 |
| Ethylene glycol distearate | 0.2 |
| Ph adjust with citric acid solution or Sodium hydroxide solution | To pH 5.2 |
| Water | To 100 |

### Example 26

Example 26 is a leave-on facial emulsion composition containing a cationic hydrophobic surfactant prepared by combining the following components utilizing conventional mixing techniques which contains fragrance I of example 9.

| **Ingredients** | **Weight %** |
|---|---|
| Water to | 100.00 |
| Glycerin | 3.00 |
| Cetyl Palmitate | 3.00 |
| Cetyl Alcohol | 1.26 |
| Quaternium-22 ** | 1.00 |
| Glyceryl Monohydroxy Stearate | 0.74 |
| Dimethicone | 0.60 |
| Stearic Acid | 0.55 |
| Octyldodecyl Myristate | 0.20 |
| Perfume I of example 9. | 0.06 |
| Carbomer 1342 * | 0.125 |
| Tetrasodium EDTA | 0.10 |
| Carbomer 951 * | 0.075 |
| *supplied by Noveon Inc Ohio USA | |
| ** supplied by Seppic, Paris, France | |

### Example 27 A sunscreen Composition

Example 27 is an oil-in-water emulsion prepared by combining the following components utilizing conventional mixing techniques which contains a fragrance G of the invention from example 7.

| **Ingredients** | **Weight %** |
|---|---|
| **Phase A** | |
| Water | 100.00 |
| Carbomer 954 (supplied by Noveon) | 0.24 |
| Carbomer 1342(supplied by Noveon) | 0.16 |
| Disodium EDTA | 0.05 |

| **Phase B** | |
|---|---|
| Isoarachidyl neopentanoate | 2.00 |
| PVP Eicosene Copolymer | 2.00 |
| Octyl methoxycinnamate | 7.50 |
| Octocrylene | 4.00 |
| Oxybenzone | 1.00 |
| Titanium Dioxide | 2.00 |
| Cetyl Palmitate | 0.75 |
| Stearoxytrimethylsilane (and Stearyl Alcohol) | 0.50 |
| Dimethycone | 1.00 |
| Tocopheryl Acetate | 0.10 |
| DEA-Cetyl Phosphate | 0.20 |

| **Phase C** | |
|---|---|
| Water | 2.00 |
| Triethanolamine 99% | 0.60 |

| **Phase D** | |
|---|---|
| Water | 2.00 |
| Perfume G of example 7 | 0.05 |
| Butylene Glycol | 2.00 |
| DMDM Hydantoin | 0.25 |
| DL Panthenol | 1.00 |

| **Ingredients** | **Weight %** |
|---|---|
| **Phase E** | |
| Cyclomethicone | 1.00 |

In a suitable vessel the Phase A ingredients are dispersed in the water and heated to about 75-85 °C. In a separate vessel the Phase B ingredients (except DEA-Cetyl Phosphate) are combined and heated to about 85-90°C until melted. Next, the DEA-Cetyl Phosphate is added to the liquid Phase B and stirred until dissolved. This mixture is then added to Phase A to form the emulsion. The Phase C ingredients are combined until dissolved and then added to the emulsion. The emulsion is then cooled to about 40-45°C with continued mixing. In another vessel, the Phase D ingredients are heated with mixing to about 40-45°C until a clear solution is formed and this solution is then added to the emulsion. Finally, the emulsion is cooled to about 35°C. and the Phase E ingredient is added and mixed.

### Example 28

Example 28 is an aqueous skin cleansing liquid for impregnating a non woven textile to form a moist wipe which contains a fragrance according to the invention.

| **Ingredient dosage** | **Weight Percentage** |
|---|---|
| Propylene Glycol | 0.8 |
| Polysorbate 20 | 1.5 |
| Germaben II (RTM) | 1.0 |
| Fragrance E of example 5 | 0.2 |
| Silicone antifoam 1510 (supplied by Univar) | 0.015 |
| Lactic acid | To pH 5.4 |
| Water | To 100% |

Germaben II is a commercial preservative from ISP. Various additives could be added to such a formulation for skin benefit such as Aloe vera, DL-panthenol, chamomile extracts in which case the water content would be adjusted to accommodate the additives. Such a liquid would be dosed at around 125gm⁻² on 50gm⁻² spunlace non woven substrate.

### Example 29

Example 29 is a phase inversion emulsion composition containing a fragrance of the invention for impregnating onto a non woven fabric as a moist wipe and incorporating a fragrance according to the invention.

| **Ingredient** | **Weight Percent** |
|---|---|
| Emulgade CM ^{®} | 15 |
| Ceteareth 20 | 4.7 |
| Dicapryl ether | 4.0 |
| Cetearyl isononanoate | 5.0 |
| Cocoglycerides | 2.0 |
| Fragrance C of example 3 | 0.15 |
| Euxyl K702 | 1.0 |
| Tetrasodium EDTA | 0.1 |
| Citric acid | 0.04 |
| Water | To 100 |
| Emulgade CM is a concentrated emulsion of cosmetic oils and non-ionic emulsifiers which dilutes into a phase inverted emulsion mixture supplied by Cognis. | |
| Ceterareth 20 is a non-ionic emulsifier. | |
| Euxyl K702 is a commercial mixture of preservatives from Schulke & Mayr containing Benzoic acid, dehydroacetic acid, phenoxyethanol, ethylhexylglycerin and polyaminopropyl biguanidine. | |

As for example 14 various beneficial additives can be incorporated into the formulation which would typically be dosed at around 125gm⁻² on 50gm⁻² spunlace non woven substrate.

### Examples 30 to 33

Examples 30-33 are dilute and concentrated Liquid fabric conditioner compositions containing a fragrance of the invention, part of which may be encapsulated and which may, but need not, have the same composition as the free fragrance.

| **Ingredient** | Example 30 Standard fabric conditionner | Example 31 Standard fabric conditionner | Example 32 Concentrated fabric conditionner | Example 33 Concentrated fabric conditionner |
|---|---|---|---|---|
| | **Wt %** | **Wt %** | **Wt %** | **wt %** |
| Tetranyl AHT-1 | 5.0 | | 12.0 | |
| Deqa | | 7.0 | | 18 |
| Genapol C200 | 0.1 | | 0.75 | 3 |
| Isopropyl alcohol | | 2.0 | | 3 |
| Polyethylene glycol 4000 | | | | 0.6 |
| Laurex CS | 0.4 | | 1.8 | |
| Encapsulated perfume F of example 6 | 0.1 | | | 0.3 |
| Free fragrance D of example 4 | 0.2 | 0.3 | 0.45 | 0.3 |
| Calcium or magnesium chloride | qs | qs | qs | qs |
| Dye, antifoaming agent, preservative | qs | qs | qs | qs |
| Water qsp | 100 | 100 | 100 | 100 |
| Tetranyl AHT-1: semi-hard tallow ester of triethanolammonium methosulphate, marketed by Kao Corp | | | | |
| Genapol C200: copra ethoxylate, marketed by Clariant | | | | |
| Laurex CS: Long chain alcohol, marketed by Albright & Wilson | | | | |
| DEQA: soft di(tallow oxyethyl) dimethylammonium chloride | | | | |

### Example 34

Example 34 is a household cleaner composition containing a fragrance of the invention.

| **Ingredient** | **Wt %** |
|---|---|
| Emulgin HF70 | 16.7 |
| 1,2 Propylene glycol | 4.0 |
| Perfume D of example 4 | 0.2 |
| Kathon CG | 0.005 |
| Natrosol 250MR | 11.7 |
| Dyes and other minors | q.s. |
| Water | To 100 |
| Emulgin HF70 supplied by Cognis KathonCG supplied by Seppic | |
| Natrosol 250MR supplied by Hercules | |

### Example 35

Example 35 is a household cleaner composition containing a fragrance of the Invention

| **Ingredient** | **Purity** | **Wt %** |
|---|---|---|
| Secondary alkane sulphonate Hostapur SAS 60 | 60 | 13.0 |
| Alcohol ethoxylate e.g. Neodol 91-6 EO | 100 | 2.0 |
| Lactic acid | 100 | 0.5 |
| Propylene glycol n butyl ether | | 0.5 |
| Perfume D of example 4 | 100 | 0.2 |
| Preservative dye other minors | | q.s. |
| Water | 100 | To 100 |
| Hostapur SAS 60 supplied by Clariant Neodol 91-6 supplied by Shell Chemicals | | |

### Example 36

Example 36 is a toilet soap bar composition containing a fragrance of the invention.

| **Ingredient** | **Wt %** |
|---|---|
| 83/17 tallow/coco soap base | 93.0 |
| Glycerin | 1.0 |
| Coconut fatty acid | 3.0 |
| Perfume C of example 3 | 1.25 |
| Titanium dioxide | 0.5 |
| EDTA | 0.1 |
| Butylated hydroxytoluene | 0.15 |
| Colourant preservatives and minors | qs |
| Water | To 100 |

### Example 37

Unfragranced shampoo base of example 25 was prepared, and fragranced with 0.2% by weight of either:
- I) a mixture 2 fragrance materials not according to the invention (ethylene brassylate, pentyl salicylate)
- II) a mixture of 5 fragrance materials according to the invention (ethylene brassylate, dihydrojasmonate, phenoxyethanol, heliotropine, ethyl vanillin)
- III) a mixture of 6 fragrance materials according to the invention (ethylene brassylate, geranyl acetate, benzyl acetate, phenoxyethanol, heliotropine, ethyl vanillin)
- IV) a conventional commercial fragrance containing more than 150 materials (fragrance used in product sold under name "Bedtime Bath" by Johnson & Johnson).
In an olfactory test using 20 subjects the fragrance I was found to be acceptable, but less preferred than fragrances II) III) and IV) which were found to be equivalent.

According to the invention this test demonstrated that it is indeed possible to create consumer liked fragrances from very few well characterized fragrance materials the use of 5 or 6 well characterized fragrance materials being superior to the use of 2.

## Claims

1. A fragrance composition for use in cosmetic, toiletry, personal care and cleansing, household cleaning and laundry products which consists of 2 to10 well characterized fragrance materials having a cosmetic function, of which are selected from: allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate, and these must comprise at least 40% by weight of the fragrance composition wherein the well characterized fragrance materials having a cosmetic function are selected from Table 1 below:
**TABLE 1: Preferred wall characterized fragrance materials having a cosmetic function**
| **Compound** | **Cas no** | **INCI name** | **Other material (%) by weight** | **Cosmetic function** |
|---|---|---|---|---|
| Acetanilide | 103-84-4 | Acetanilid | Less than 1% | Stabilising |
| Pentyl salicylate | 2050-08-0 | Amyl salicylate | Less than 1% | Skin conditionner |
| Hexanol | 111-27-3 | Hexyl alcohol | Less than 1% | Antifoaming/solvent/ hydrotrope |
| Octanol | 111-87-5 | Caprylic alcohol | Less than 1% | Viscosity controlling |
| Aldehyde C-14 (undecalacione gamma) | 104-67-6 | Gamma-undecalactone | Less than 1% | Solvent/masking |
| Aldehyde C-16 (Ethyl methyl phenyl glycidate) | 77-83-8 | Ethyl methylphenyl Glyddate | Less than 1% | Viscosity controlling |
| Aldehyde C-18 (nonalactone.gamma) | 104-61-0 | Gamma-nonalactone | Less than 1% | Solvent |
| Allyl caproate | 123-68-2 | Allyl caproate | Less than 1% | Emollient |
| Amyl acetate | 628-63-7 | Amyl acetate | Less than 1% | Solvent |
| Amyl benzoate | 2049-96-0 | Amyl benzoate | Less than 1% | Solvent/masking |
| Benzyl acetate | 140-11-4 | Benzyl acetate | Less than 1% | Solvent |
| Benzaldehyde | 100-52-7 | Benzaldehyde | Less than 1% | Solvent |
| Benzyl salicylate | 118-58-1 | Benzyl salicylate | Less than 1% | UV absorber |
| Benzyl alcohol | 100-51-6 | Benzyl alcohol | Less than 5% | Preservative/solvent |
| Isopropyl myristate | 11-21-0 | Isopropyl myristate | Less than 5% | Binding/emollient/ solvent/skin conditioner |
| Citronellol | 106-22-9 | Citronellol | Less than 5% | Masking |
| Citronellyl acetate | 150-84-5 | Citronellyl acetate | Less than 5% | Masking |
| Costauton^{®} | 67770-79-0 | Ethyl butylvalerolactone | Less than 1% | Tonic |
| Coumarin | 91-64-5 | Coumarin | Less than 1% | Masking |
| Cyclamen aldehyde | 103-95-7 | Cyclamen aldehyde | Less than 1% | Masking |
| Cyclopentadecanolide | 106-02-5 | Pentadecalactone | Less than 1% | Masking |
| Decalactone | 705-86-2 | Delta-decalactone | Less than 1% | Masking |
| Decanal | 112-31-2 | Decanal | Less than 5% | Masking |
| Dihydro isojasmonate | 37172-53-5 | Dihydrojasmonate | Less than 1% | Tonic |
| Dipropylene glycol | 25265-71-8 | Dipropylene glycol | Less than 10% | Solvent |
| Efetaal^{®} | 2556-10-7 | Ethyl phenethylacetat | Less than 1% | Solvent/tonic |
| Ethyl dnnamate | 103-36-6 | Ethyl cinnamate | Less than 1% | UV absorber |
| Ethyl laurate | 106-33-2 | Ethyl laurate | Less than 1% | Emollient |
| Ethyl pelargonate | 123-29-5 | Ethyl pelargonate | Less than 1% | Emollient |
| Ethyl vanillin | 121-32-4 | Ethyl vanillin | Less than 1% | Soothing/masking |
| Fleuramone^{®} | 137-03-1 | 2-Heptylcyclopentanone | Less than 1% | Solvent |
| Geraniol | 106-24-1 | Geraniol | Less than 1% | Tonic |
| Geranyl acetate | 105-87-3 | Geranyl acetate | Less than 5% | Tonic |
| Geranyl acetate | 105-87-3 | Geranyl acetate | Less than 5% | Tonic |
| Heliotropine | 120-57-0 | Heliotropine | Less than 1% | Masking/skin conditioning |
| Cis hex-3-en-1-ol | 928-96-1 | 3-Hexenol | Less than 1% | Solvent/humectant/ viscosity controlling |
| Hydroxycitronellat | 107-75-5 | Hydroxycitronellal | Less than 1% | Masking |
| Iso butyl benzoate | 120-50-3 | Isobutyl benzoate | Less than 1% | Preservative/solvent |
| Linalool | 78-70-6 | Linalool | Less than 5% | Deodorant |
| Linalyl acetate | 115-05-7 | Linalyl acetate | Less than 5% | Masking |
| Menthyl acetate | 89-48-5 | Menthyl acetate | Less than 1% | Refreshing/masking |
| Methyl dihydrojasmonate | 2630-39-9 | Methyldihydro-jasmonate | Less than 1% | Masking |
| Methyl salicylate | 119-36-8 | Methyl salicylate | Less than 1% | Denaturing/soothing |
| Ethylene brassytate | 105-95-3 | Ethylene brassylate | Less than 10% | Tonic/masking |
| Phenoxanol | 55066-48-3 | Phenylisohexanol | Less than 1% | Masking |
| Phenoxyethanol | 122-99-6 | Phenoxyethanol | Less than 1% | Preservative |
| Phenyl salicylate | 118-55-8 | Phenyl salicytate | Less than 5% | Antimicrobial |
| Triacetin | 102-16-1 | Triacetin | Less than 1% | Antimicrobial/film forming /solvent |
| Propyl acetate | 109-60-4 | Propyl acetate | Less than 1% | Solvent |
| Santalex T^{®} | 68877-29-2 | Camphylcyclohexanol | Less than 10% | Solvent |
| Triethyl citrate | 77-93-0 | Triethyl citrate | Less than 1% | Antioxidant/ deodorant/solvent |
| Vanillin | 121-33-5 | Vanillin | Less than 1% | Masking |
| Verdox^{®} | 88-41-5 | 2-t-butyl-cyclohexylacetate | Less than 1% | Solvent |
| 2-Phenyl ethanol | 60-12-8 | Phenylethanol | Less than 5% | Deodorant |
| 2-Phenylethyl acetate | 103-45-7 | Phenyl ethyl acetate | Less than 5% | Deodorant |
| Anethol | 104-46-1 | Anethol | Less than 1% | Denaturant |
| Benzophenone | 119-61-9 | Benzophenone | Less than 1% | UV absorber |
| Citronellal | 106-23-0 | Citronellal | Less than 5% | Masking agent |
| Decenal | 65405-70-1 | Decenal | Less than 5% | Masking agent |
| **Compound** | **Cas no** | **INCI name** | **Other material(%) by weight** | **Cosmetic function** |
|---|---|---|---|---|
| Methyl anthranilate | 134-20-3 | Methyl anthranylate | Less than 1% | Masking |
| Paracymene | 99-87-6 | P-cymene | Less than 5% | Masking |
| Terpineol | 98-55-5 | Terpineol | Less than 10% | Denaturant/solvent |
| Hinokitiol | 499-44-5 | Hinokitiol | Less than 1% | Antistatichair conditioning |

2. The fragrance composition according to claims 1, wherein the fragrance composition is encapsulated.

3. The fragrance composition according to claim 2, wherein the fragrance composition is encapsulated within a water soluble spray dried starch based capsule.

4. The fragrance composition according to claim 2, wherein the fragrance composition is encapsulated within a water insoluble aminoplast capsule.

5. Use of a fragrance composition according to any one of claims 1 to 4, for the manufacture of a leave on personal care composition, optionally including mixtures of free and encapsulated fragrance.

6. Use of a fragrance composition according to any one of claims 1 to 4, for the manufacture of a rinse off personal cleansing composition comprising a detersive ingredient, optionally including mixtures of free and encapsulated fragrance.

7. Use of a fragrance composition according to any one of claims 1 to 4, for the manufacture of a laundry composition comprising a detersive ingredient or softening or conditioning agent, optionally including mixtures of free and encapsulated fragrance.

8. Use of a fragrance composition according to any of claims 1 to 4, for the manufacture of a household cleaning or freshening composition comprising a detersive ingredient optionally including mixtures of free and encapsulated fragrance.

9. Use according to any one of claims 5 to 8, in which the leave on personal care composition, the rinse off personal cleansing composition, the laundry composition or the household cleaning or freshening composition contains at least 15% by weight of water.

## Patentansprüche

1. Duftstoffzusammensetzung zur Verwendung in Kosmetik-, Toilettenartikel-, Körperpflege- und -reinigungs-, Haushaltsreinigungs- und Waschmittelprodukten, die aus 2 bis 10 gut charakterisierten Duftstoffen mit einer kosmetischen Funktion besteht, von denen wenigstens 4 aus Allylcaproat, Benzylacetat, Benzaldehyd, Dihydroisojasmonat, Ethylcinnamat, Ethylmethylphenylglycidat, Ethylvanillin, Geranylacetat, Heliotropin, cis-Hex-3-en-1-ol, Ethylenbrassylat, gamma-Nonalacton, Camphylcyclohexanol, gamma-Undecalacton, Pentylsalicylat, 2-t-Butylcyclohexylacetat ausgewählt sind,
und diese wenigstens 40 Gewichts-% der Duftstoffzusammensetzung ausmachen müssen,
wobei die gut charakterisierten Duftstoffe mit einer kosmetischen Funktion aus der folgenden Tabelle 1 ausgewählt sind:
**TABELLE 1: Bevorzugte gut charakterisierte Duftstoffe mit einer kosmetischen Funktion**
| **Verbindung** | **Cas Nr.** | **INCI Name** | **Gewicht anderer Materialien (%)** | **Kosmetische Funktion** |
|---|---|---|---|---|
| Acetanilid | 103-84-4 | Acetanilid | weniger als 1 % | Stabilisierung |
| Pentylsalicylat | 2050-08-0 | Amylsalicylat | weniger als 1 % | Hautkonditioner |
| Hexanol | 111-27-3 | Hexylalkohol | weniger als 1 % | antischäumend/ Lösungsmittel/ hydrotrop |
| Octanol | 111-87-5 | Caprylalkohol | weniger als 1 % | Viskositätskontrolle |
| Aldehyd C-14 (gamma-Undecalacton) | 104-67-6 | gamma-Undecalacton | weniger als 1 % | Lösungsmittel/ Abdecken |
| Aldehyd C-16 (Ethylmethylphenylglycidat) | 77-83-8 | Ethylmethylphenylglycidat | weniger als 1 % | Viskositätskontrolle |
| Aldehyde C-18 (gamma-Nonalacton) | 104-61-0 | gamma-Nonalacton | weniger als 1 % | Lösungsmittel |
| Allylcaproat | 123-68-2 | Allylcaproat | weniger als 1 % | Emolliens |
| Amylacetat | 628-63-7 | Amylacetat | weniger als 1 % | Lösungsmittel |
| Amylbenzoat | 2049-96-9 | Amylbenzoat | weniger als 1 % | Lösungsmittel/ Abdecken |
| Benzylacetat | 140-11-4 | Benzylacetat | weniger als 1 % | Lösungsmittel |
| Benzaldehyd | 100-52-7 | Benzaldehyd | weniger als 1 % | Lösungsmittel |
| Benzylsalicylat | 118-58-1 | Benzylsalicylat | weniger als 1 % | UV Absorbierer |
| Benzylalkohol | 100-51-6 | Benzylalkohol | weniger als 5 % | Konservierungsmittel/ Lösungsmittel |
| Isopropylmyristat | 11-21-0 | Isopropylmyristat | weniger als 5 % | Bindemittel/ Emolliens/ Lösungsmittel/ Hautkonditioner |
| Citronellol | 106-22-9 | Citronellol | weniger als 5 % | Abdecken |
| Citronellylacetat | 150-84-5 | Citronellylacetat | weniger als 5 % | Abdecken |
| Costaulon^{®} | 67770-79-0 | Ethylbutylvalerolacton | weniger als 1 % | Tonic |
| Cumarin | 91-64-5 | Cumarin | weniger als 1 % | Abdecken |
| Cyclamenaldehyd | 103-95-7 | Cyclamenaldehyd | weniger als 1 % | Abdecken |
| Cyclopentadecanolid | 106-02-5 | Pentadecalacton | weniger als 1 % | Abdecken |
| Decalacton | 705-86-2 | delta-Decalacton | weniger als 1 % | Abdecken |
| Decanal | 112-31-2 | Decanal | weniger als 5 % | Abdecken |
| Dihydroisojasmonat | 37172-53-5 | Dihydrojasmonat | weniger als 1 % | Tonic |
| Dipropylenglycol | 25265-71-8 | Dipropylenglycol | weniger als 10% | Lösungsmittel |
| Efetaal^{®} | 2556-10-7 | Ethylphenethylacetal | weniger als 1 % | Lösungsmittel/ Tonic |
| Ethylcinnamat | 103-36-6 | Ethylcinnamat | weniger als 1 % | UV Absorbierer |
| Ethyllaurat | 106-33-2 | Ethyllaurat | weniger als 1 % | Emolliens |
| Ethylpelargonat | 123-29-5 | Ethylpelargonat | weniger als 1 % | Emolliens |
| Ethylvanillin | 121-32-4 | Ethylvanillin | weniger als 1 % | Linderung/ Abdecken |
| Fleuramone^{®} | 137-03-1 | 2-Heptylcyclopentanon | weniger als 1 % | Lösungsmittel |
| Geraniol | 106-24-1 | Geraniol | weniger als 1 % | Tonic |
| Geranylacetat | 105-87-3 | Geranylacetat | weniger als 5 % | Tonic |
| Geranylacetat | 105-87-3 | Geranylacetat | weniger als 5 % | Tonic |
| Heliotropin | 120-57-0 | Heliotropin | weniger als 1 % | Abdecken/ Hautkonditioner |
| cis-Hex-3-en-1-ol | 928-96-1 | 3-Hexenol | weniger als 1 % | Lösungsmittel/ Feuchthaltemittel/ Viskositätskontrolle |
| Hydroxycitronellal | 107-75-5 | Hydroxycitronellal | weniger als 1 % | Abdecken |
| Isobutylbenzoat | 120-50-3 | Isobutylbenzoat | weniger als 1 % | Konservierungsmittel/ Lösungsmittel |
| Linalool | 78-70-6 | Linalool | weniger als 5 % | Deodorant |
| Linalylacetat | 115-95-7 | Linalylacetat | weniger als 5 % | Abdecken |
| Menthylacetat | 89-48-5 | Menthylacetat | weniger als 1 % | Erfrischen/ Abdecken |
| Methyldihydrojasmonat | 2630-39-9 | Methyldihydrojasmonat | weniger als 1 % | Abdecken |
| Methylsalicylat | 119-36-8 | Methylsalicylate | weniger als 1 % | Denaturierung/ Linderung |
| Ethylenbrassylat | 105-95-3 | Ethylenbrassylat | weniger als 10% | Tonic/ Abdecken |
| Phenoxanol | 55066-48-3 | Phenylisohexanol | weniger als 1 % | Abdecken |
| Phenoxyethanol | 122-99-6 | Phenoxyethanol | weniger als 1 % | Konservierungsmittel |
| Phenylsalicylat | 118-55-8 | Phenylsalicylat | weniger als 5 % | antimikrobiell |
| Triacetin | 102-76-1 | Triacetin | weniger als 1 % | antimikrobiell/ filmbildend/ Lösungsmittel |
| Propylacetat | 109-60-4 | Propylacetat | weniger als 1 % | Lösungsmittel |
| Santalex T^{®} | 68877-29-2 | Camphylcyclohexanol | weniger als 10% | Lösungsmittel |
| Triethylcitrat | 77-93-0 | Triethylcitrat | weniger als 1 % | Antioxidans/ Deodorant/ Lösungsmittel |
| Vanillin | 121-33-5 | Vanillin | weniger als 1 % | Abdecken |
| Verdox^{®} | 88-41-5 | 2-t-Butylcyclohexylacetat | weniger als 1 % | Lösungsmittel |
| 2-Phenylethanol | 60-12-8 | Phenylethanol | weniger als 5 % | Deodorant |
| 2-Phenylethylacetat | 103-45-7 | Phenylethylacetat | weniger als 5 % | Deodorant |
| Anethol | 104-46-1 | Anethol | weniger als 1 % | Denaturierungsmittel |
| Benzophenon | 119-61-9 | Benzophenon | weniger als 1 % | UV Absorbierer |
| Citronellal | 106-23-0 | Citronellal | weniger als 5 % | Abdeckmittel |
| Decenal | 65405-70-1 | Decenal | weniger als 5 % | Abdeckmittel |
| Methylanthranilat | 134-20-3 | Methylanthranylat | weniger als 1 % | Abdecken |
| Paracymen | 99-87-6 | P-Cymen | weniger als 5 % | Abdecken |
| Terpineol | 98-55-5 | Terpineol | weniger als 10 % | Denaturierungsmittel/ Lösungsmittel |
| Hinokitiol | 499-44-5 | Hinokitiol | weniger als 1 % | antistatisch/ Haarkonditioner |

2. Duftstoffzusammensetzung nach Anspruch 1, wobei die Duftstoffzusammensetzung eingekapselt ist.

3. Duftstoffzusammensetzung nach Anspruch 2, wobei die Duftstoffzusammensetzung in einer wasserlöslichen, sprühgetrockneten, stärkebasierten Kapsel eingekapselt ist.

4. Duftstoffzusammensetzung nach Anspruch 2, wobei die Duftstoffzusammensetzung in einer wasserunlöslichen Aminoplastkapsel eingekapselt ist.

5. Verwendung einer Duftstoffzusammensetzung nach einem der Ansprüche 1 bis 4 für die Herstellung einer Körperpflegezusammensetzung zum Auftragen, die wahlweise Mischungen freier und eingekapselter Duftstoffe umfasst.

6. Verwendung einer Duftstoffzusammensetzung nach einem der Ansprüche 1 bis 4 für die Herstellung einer abzuspülenden Körperpflegezusammensetzung, die einen Waschmittelbestandteil umfasst und die wahlweise Mischungen freier und eingekapselter Duftstoffe umfasst.

7. Verwendung einer Duftstoffzusammensetzung nach einem der Ansprüche 1 bis 4 für die Herstellung einer Waschmittelzusammensetzung, die einen Waschmittelbestandteil oder ein Weichmacher- oder Konditionermittel umfasst und die wahlweise Mischungen freier und eingekapselter Duftstoffe umfasst.

8. Verwendung einer Duftstoffzusammensetzung nach einem der Ansprüche 1 bis 4 für die Herstellung einer Haushaltsreinigungs- oder -auffrischzusammensetzung, die einen Waschmittelbestandteil umfasst und die wahlweise Mischungen freier und eingekapselter Duftstoffe umfasst.

9. Verwendung nach einem der Ansprüche 5 bis 8, in der die Körperpflegezusammensetzung zum Auftragen, die abzuspülende Körperpflegezusammensetzung, die Waschmittelzusammensetzung oder die Haushaltsreinigungs- oder -auffrischzusammensetzung wenigstens 15 Gewichts-% Wasser umfasst.

## Revendications

1. Composition de parfum pour utilisation dans des produits cosmétiques, de toilette, de soin personnel, nettoyants, ménagers ou détergents, qui consiste en 2 à 10 matières de parfum bien caractérisées ayant une fonction cosmétique, dont au moins 4 sont choisis parmi :
caproate d'allyle, acétate de benzyle, benzaldéhyde, dihydroisojasmonate, cinnamate d'éthyle, méthylphényl glycidate d'éthyle, éthylvanilline, acétate de géranyle, héliotropine, cis hex-3-èn-1-ol, éthylène brassylate, nonalactone gamma, camphylcyclohexanol, undécalactone gamma, salicylate de pentyle et acétate de 2-t-butylcyclohexyle, qui doivent représenter au moins 40% en poids de la composition de parfum ;
dans laquelle les matières de parfum bien caractérisées ayant une fonction cosmétique sont choisies dans le tableau 1 ci-dessous :
**Tableau 1: matières de parfum bien caractérisées ayant une fonction cosmétique préférées**
| **composé** | **N° Cas** | **Nom INCI** | **Autre matière (% en poids)** | **Fonction cosmétique** |
|---|---|---|---|---|
| Acétanilide | 103-84-4 | Acétanilide | Moins de 1 % | Stabilisant |
| Salicylate de pentyle | 2050-08-0 | Salicylate d'amyle | Moins de 1 % | Conditionneur de peau |
| Hexanol | 111-27-3 | Alcool hexylique | Moins de 1 % | Antimousse/solvant/ hydrotrope |
| Octanol | 111-87-5 | Alcool caprylique | Moins de 1 % | Contrôle de la viscosité |
| Aldéhyde C-14 (undécalactone gamma) | 104-67-6 | Gamma-undécalactone | Moins de 1 % | Solvant/masquant |
| Aldéhyde C-16 (méthylphénylglycid-ate d'éthyle) | 77-83-8 | Méthylphénylglycid-ate d'éthyle | Moins de 1 % | Contrôle de la viscosité |
| Aldéhyde C-18 (nonalactone, gamma) | 104-61-0 | Gamma-nonalactone | Moins de 1 % | Solvant |
| Caproate d'allyle | 123-68-2 | Caproate d'allyle | Moins de 1 % | Emollient |
| Acétate d'amyle | 628-63-7 | Acétate d'amyle | Moins de 1 % | Solvant |
| Benzoate d'amyle | 2049-96-9 | Benzoate d'amyle | Moins de 1 % | Solvant/masquant |
| Acétate de benzyle | 140-11-4 | Acétate de benzyle | Moins de 1 % | Solvant |
| Benzaldéhvde | 100-52-7 | Benzaldéhvde | Moins de 1 % | Solvant |
| Salicylate de benzyle | 118-58-1 | Salicylate de benzyle | Moins de 1 % | Absorbant UVr |
| Alcool benzvlique | 100-51-6 | Alcool benzylique | Moins de 5 % | Conservateur/solvant |
| Myristate d'isopropyle | 11-21-0 | Myristate d'isopropyle | Moins de 5 % | Liant/émollient/ solvant/ conditionneur de peau |
| Citronellol | 106-22-9 | Citronellol | Moins de 5 % | Masquant |
| Acétate de citronellyle | 150-84-5 | Acétate de citronellyle | Moins de 5 % | Masquant |
| Costaulon ^{®} | 67770-79-0 | Ethylbutylvalérolactone | Moins de 1 % | Tonique |
| Coumarine | 91-64-5 | Coumarine | Moins de 1 % | Masquant |
| Aldéhyde cyclamen | 103-95-7 | Aldéhyde cyclamen | Moins de 1 % | Masquant |
| Cyclopentadécanolid e | 106-02-5 | Pentadécalactone | Moins de 1 % | Masquant |
| Décalactone | 705-86-2 | Delta-décalactone | Moins de 1 % | Masquant |
| Décanal | 112-31-2 | Décanal | Moins de 5 % | Masquant |
| Dihydroisojasmonate | 37172-53-5 | Dihydrojasmonate | Moins de 1 % | Tonique |
| Dipropylène glycol | 25265-71-8 | Dipropylène glycol | Moins de 10 % | Solvant |
| Efetaal ^{®} | 2556-10-7 | phénéthylacétal éthylique | Moins de 1 % | Solvant/tonique |
| Cinnamate d'éthyle | 103-36-6 | Cinnamate d'éthyle | Moins de 1 % | Absorbant UV |
| Laurate d'éthyle | 106-33-2 | Laurate d'éthyle | Moins de 1% | Emollient |
| Pélargonate d'éthyle | 123-29-5 | Pélargonate d'éthyle | Moins de 1 % | Emollient |
| Ethyl vanilline | 121-32-4 | Ethyl vanilline | Moins de 1 % | Apaisant/masquant |
| Fleuramone ^{®} | 137-03-1 | 2-Heptylcyclopentanone | Moins de 1 % | Solvant |
| Géraniol | 106-24-1 | Géraniol | Moins de 1 % | Tonique |
| Acétate de géranyle | 105-87-3 | Acétate de géranyle | Moins de 5% | Tonique |
| Héliotropine | 120-57-0 | Héliotropine | Moins de 1 % | Masquant/conditionneur de peau |
| Cis hex-3-èn-1-ol | 928-96-1 | 3-Hexenol | Moins de 1 % | Solvant/humectant/ contrôle de la viscosité |
| Hydroxycitronellal | 107-75-5 | Hydroxycitronellal | Moins de 1 % | Masquant |
| Benzoate d'isobutyle | 120-50-3 | Benzoate d'isobutyle | Moins de 1 % | Conservateur/solvant |
| Linalool | 78-70-6 | Linalool | Moins de 5% | Déodorant |
| Acétate de linalyle | 115-95-7 | Acétate de linalyle | Moins de 5% | Masquant |
| Acétate de menthyle | 89-48-5 | Acétate de menthyle | Moins de 1 % | Rafraîchissant/ masquant |
| Dihydrojasmonate de méthyle | 2630-39-9 | Dihydrojasmonate de méthyle | Moins de 1 % | Masquant |
| Salicylate de méthyle | 119-36-8 | Salicylate de méthyle | Moins de 1 % | Dénaturant/apaisant |
| Ethylène brassylate | 105-95-3 | Ethylène brassylate | Moins de 10% | Tonique/masquant |
| Phénoxanol | 55066-48-3 | Phénylisohexanol | Moins de 1 % | Masquant |
| Phénoxyéthanol | 122-99-6 | Phénoxyéthanol | Moins de 1 % | Conservateur |
| Salicylate de phényle | 118-55-8 | Salicylate de phényle | Moins de 5% | Antimicrobien |
| Triacétine | 102-76-1 | Triacétine | Moins de 1 % | Antimicrobien/ filmogène/solvant |
| Acétate de propyle | 109-60-4 | Acétate de propyle | Moins de 1 % | Solvant |
| Santalex T^{®} | 68877-29-2 | Camphylcyclohexanol | Moins de 10 % | Solvant |
| Citrate de triéthyle | 77-93-0 | Citrate de triéthyle | Moins de 1 % | Antioxydant/ déodorant/solvant |
| Vanilline | 121-33-5 | Vanilline | Moins de 1 % | Masquant |
| Verdox^{®} | 88-41-5 | Acétate de 2-t-butyl-cyclohexyle | Moins de 1 % | Solvant |
| 2-Phényléthanol | 60-12-8 | Phényléthanol | Moins de 5 % | Déodorant |
| Acétate de 2-phényléthyle | 103-45-7 | Acétate de 2-phényléthyle | Moins de 5 % | Déodorant |
| Anéthol | 104-46-1 | Anéthol | Moins de 1 % | Dénaturant |
| Benzophénone | 119-61-9 | Benzophénone | Moins de 1 % | Absorbant UV |
| Citronellal | 106-23-0 | Citronellal | Moins de 5 % | Masquant |
| Décénal | 65405-70-1 | Décenal | Moins de 5 % | Masquant |
| Anthranilate de méthyle | 134-20-3 | Anthranilate de méthyle | Moins de 1 % | Masquant |
| Paracymène | 99-87-6 | P-cymène | Moins de 5 % | Masquant |
| Terpinéol | 98-55-5 | Terpinéol | Moins de 10 % | Dénaturant/solvant |
| Hinokitiol | 499-44-5 | Hinokitiol | Moins de 1 % | Antistatique/ conditionneur pour cheveux |

2. Composition de parfum selon la revendication 1, qui est encapsulée.

3. Composition de parfum selon la revendication 2, qui est encapsulée dans une capsule hydrosoluble à base d'amidon séché par pulvérisation.

4. Composition de parfum selon la revendication 2, qui est encapsulée dans une capsule aminoplaste insoluble dans l'eau.

5. Utilisation d'une composition de parfum selon l'une quelconque des revendications 1 à 4 pour la fabrication d'une composition de soin personnelle à ne pas rincer, qui inclut éventuellement des mélanges de parfum libre et de parfum encapsulé.

6. Utilisation d'une composition de parfum selon l'une quelconque des revendications 1 à 4 pour la fabrication d'une composition de nettoyage personnelle à rincer comprenant un ingrédient détersif, qui inclut éventuellement des mélanges de parfum libre et de parfum encapsulé.

7. Utilisation d'une composition de parfum selon l'une quelconque des revendications 1 à 4 pour la fabrication d'une composition détergente comprenant un ingrédient détergent, un agent adoucissant ou un agent assouplissant, qui inclut éventuellement des mélanges de parfum libre et de parfum encapsulé.

8. Utilisation d'une composition de parfum selon l'une des revendications 1 à 4 pour la fabrication d'une composition rafraîchissante ou de nettoyage ménager comprenant un ingrédient détergent, qui inclut éventuellement des mélanges de parfum libre et de parfum encapsulé.

9. Utilisation selon l'une quelconque des revendications 5 à 8, dans laquelle la composition de soin personnelle à ne pas rincer, la composition de nettoyage personnelle à rincer, la composition détergente ou la composition rafraîchissante ou de nettoyage ménager contient au moins 15% en masse d'eau.
